(19) Europäisches Patentamt / European Patent Office / Office européen des brevets

(11) **EP 4 029 533 A1**

(12) **EUROPEAN PATENT APPLICATION**
published in accordance with Art. 153(4) EPC

(43) Date of publication:
**20.07.2022 Bulletin 2022/29**

(21) Application number: **19957934.3**

(22) Date of filing: **25.12.2019**

(51) International Patent Classification (IPC):
**A61L 9/01** (2006.01)      **A61L 9/014** (2006.01)
**B01J 23/72** (2006.01)      **B01J 29/08** (2006.01)

(52) Cooperative Patent Classification (CPC):
**A61L 9/01; A61L 9/014; B01J 23/72; B01J 29/08**

(86) International application number:
**PCT/JP2019/050962**

(87) International publication number:
**WO 2021/130927 (01.07.2021 Gazette 2021/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Nikki-Universal Co., Ltd.**
**Shinagawa-ku**
**Tokyo 141-8563 (JP)**

(72) Inventor: **TAKENOUCHI Kenta**
**Hiratsuka-shi, Kanagawa 254-0014 (JP)**

(74) Representative: **Jones, Nicholas Andrew**
**Withers & Rogers LLP**
**2 London Bridge**
**London SE1 9RA (GB)**

(54) **DEODORIZING CATALYST, SLURRY FOR FORMING DEODORIZING CATALYST, DEODORIZING CATALYST STRUCTURE, METHOD FOR PRODUCING DEODORIZING CATALYST STRUCTURE AND DEODORIZATION METHOD**

(57)     The present invention relates a deodorizing catalyst including a copper-manganese-based composite oxide, zeolite, and activated carbon.

EP 4 029 533 A1

**Description**

**Technical Field**

The present invention relates to a deodorizing catalyst, a slurry for forming a deodorizing catalyst, a deodorizing catalyst structure, a method for producing a deodorizing catalyst, and a deodorization method.

**Background Art**

As a catalyst for adsorbing unpleasant sulfur-based malodorous gases such as methyl mercaptan and ethyl mercaptan, there is known a catalyst containing activated manganese dioxide and high-silica zeolite as active ingredients (e.g., Patent Literature 1).

**Citation List**

**Patent Literature**

Patent Literature 1: Japanese Unexamined Patent Publication No. H10-137591

**Summary of Invention**

Incidentally, examples of such malodorous gases in houses and other buildings include amine-based compounds such as trimethylamine, in addition to the sulfur compounds as described above. However, it is currently difficult to satisfactorily remove both the compounds with conventional catalysts including the catalyst of Patent Literature 1 described above, for example.

**Technical Problem**

The present invention has been made in view of the above circumstances, and it is an object thereof to provide a deodorizing catalyst which is excellent in removability of a sulfur compound and an amine-based compound. It is another object to provide a slurry for forming a deodorizing catalyst, a deodorizing catalyst structure, a method for producing a deodorizing catalyst, and a deodorization method.

**Solution to Problem**

The present invention provides a deodorizing catalyst including a copper-manganese-based composite oxide, zeolite, and activated carbon. The inventor has found that a deodorizing catalyst capable of achieving deodorizing performance of both of a sulfur compound and an amine-based compound is provided by mixing these three components.
Examples of the sulfur compound and examples of the amine-based compound include methyl mercaptan and trimethylamine, respectively. Zeolite is excellent in removal performance on trimethylamine, but is not sufficient in removal performance on methyl mercaptan. In contrast, the copper-manganese-based composite oxide is excellent in removal performance on methyl mercaptan, but is not sufficient in removal performance on trimethylamine. In consideration of the above, a mixture of the copper-manganese-based composite oxide and zeolite is expected to be able to achieve removal performance of both trimethylamine and methyl mercaptan, but it has been found that it is difficult to actually achieve removal performance of both trimethylamine and methyl mercaptan at a practically sufficient level. However, it was unexpectedly possible to achieve removal performance of both trimethylamine and methyl mercaptan at a practically sufficient level by adding activated carbon to this mixture. Activated carbon is generally superior to the copper-manganese-based composite oxide in terms of removal performance on trimethylamine but is inferior to zeolite. Accordingly, the removal performance of trimethylamine is expected to decrease as the proportion of activated carbon in the catalyst increases, but it has been found that the removal performance is rather slightly improved when the copper-manganese-based composite oxide coexists. In addition, the removal performance of activated carbon on methyl mercaptan is almost equivalent to that of zeolite and cannot be said to be sufficient. Accordingly, even when activated carbon is added, the removal performance on methyl mercaptan cannot be expected to be improved, but it has been found that the removal performance is actually improved. As described above, the inventor has confirmed that an effect that cannot be expected by those skilled in the art is developed in the combination of three components: a copper-manganese-based composite oxide; zeolite; and activated carbon.
The present invention may include 10 to 50% by mass of a copper-manganese-based composite oxide, 10 to 75% by mass of the zeolite, and 10 to 75% by mass of activated carbon based on the total amount of the deodorizing catalyst.

In the present invention, the silica/alumina molar ratio of the zeolite may be 100 or less.

In the present invention, the cationic species of the zeolite may be hydrogen ions.

In the present invention, the specific surface area of the activated carbon may be 500 $m^2$/g or more.

The present invention may be used for odors containing a sulfur compound and an amine-based compound.

The present invention also provides a deodorizing catalyst structure including a substrate and a catalyst layer containing the above deodorizing catalyst on the substrate.

The present invention also provides a deodorization method including a step of bringing the above deodorizing catalyst or the above deodorizing catalyst structure into contact with an odor containing a sulfur compound and an amine-based compound.

The present invention also provides a slurry for forming a deodorizing catalyst including a copper-manganese-based composite oxide, zeolite, activated carbon, and liquid component.

The present invention also provides a method for producing a deodorizing catalyst structure, the method including a step of forming a catalyst layer on a substrate using the above slurry.

**Advantageous Effects of Invention**

According to the present invention, it is possible to provide a deodorizing catalyst which is excellent in removability of a sulfur compound and an amine-based compound. According to the present invention, it is also possible to provide a slurry for forming a deodorizing catalyst, a deodorizing catalyst structure, a method for producing a deodorizing catalyst, and a deodorization method. The phrase "excellent in removability of a sulfur compound and an amine-based compound" described above does not necessarily mean that the numerical values of removability of the both compounds are high. The phrase includes, for example, maintaining good removability of one compound in addition to being excellent in removability of the other one.

**Description of Embodiments**

Hereinafter, preferred embodiments of the present invention will be described in detail, but the present invention is not limited to the following embodiments.

<Deodorizing Catalyst>

The deodorizing catalyst includes a copper-manganese-based composite oxide, zeolite, and activated carbon. Such a deodorizing catalyst can remove a sulfur compound and an amine-based compound in an extremely well-balanced manner. Examples of the sulfur compound include methyl mercaptan, ethyl mercaptan, and hydrogen sulfide. Examples of the amine-based compound include methylamine, dimethylamine, and trimethylamine. It can be said that such a deodorizing catalyst is for odors including a sulfur compound and an amine-based compound. It can be said that, among them, the above deodorizing catalyst is particularly suitable for odors including methyl mercaptan and trimethylamine and is for odors including methyl mercaptan and trimethylamine.

(Copper-manganese-based Composite Oxide)

A copper-manganese-based composite oxide is a higher-order oxide composed of an oxide containing copper and manganese. The copper-manganese-based composite oxide, which is prepared by a co-precipitation method or the like using a manganese raw material and a copper raw material, exhibits an X-ray diffraction pattern different from that of manganese oxide or copper oxide, and thus is considered to be different from a simple mixture of manganese oxide and copper oxide. The copper-manganese-based composite oxide includes copper and manganese in an amount of, for example, 10 to 70% by mass and 30 to 90% by mass, respectively, in terms of copper oxide (CuO) and manganese oxide ($MnO_2$). The copper-manganese-based composite oxide may have a hopcalite structure. The content of each component can be determined by X-ray fluorescence (XRF) analysis or high frequency inductively coupled plasma (ICP) emission spectrometry.

The metal element constituting the copper-manganese-based composite oxide can be only manganese and copper, but may contain other metal elements such as potassium, sodium, calcium, cobalt, and silver as necessary.

The copper-manganese-based composite oxide may have any suitable shape and may be granular. In the case of being granular, the average particle diameter can be from 0.1 to 50 $\mu$m and may be from 5 to 20 $\mu$m. The average particle diameter (integrated volume percentage D50) can be measured by a laser diffraction/scattering method.

The content of the copper-manganese-based composite oxide may be from 10 to 50% by mass, from 12 to 40% by mass, or from 14 to 30% by mass based on the total amount of the deodorizing catalyst in order for the catalyst to exhibit optimum deodorizing performance.

(Zeolite)

The crystal type of the zeolite is not particularly limited, and examples thereof include an X type, a Y type, a mordenite type, a ZSM-5 type, and a combination of these types. By use of zeolite having a pore size that is large to some extent, a high desorbability can be expected when cycles of adsorption and regeneration are repeated. Among them, the Y type is preferable from the viewpoint of cost. The cationic species of the zeolite may be hydrogen ions. For example, sodium ions are also conceivable as the cationic species, but hydrogen ions are more excellent. Thus, it is presumed that the Bronsted acid site contributes to removal of the amine-based compound.

When the silica/alumina molar ratio, which is the ratio between the Si element and the Al element contained in the zeolite, is smaller, the removal performance of the amine-based compound is more excellent. Thus, it is presumed that the acid amount of the zeolite is more important than the acid strength thereof. The silica/alumina molar ratio can be 100 or less and may be 30 or less. The lower limit of the silica/alumina molar ratio is not particularly limited, but can be, for example, 3. It can be said that zeolite having a small silica/alumina molar ratio is hydrophilic zeolite. Since the amine-based compound such as trimethylamine contained in odors is soluble in water, hydrophilic zeolite, when containing water vapor in the air, adsorbs such an amine-based compound easily.

The zeolite may have any suitable shape and may be granular. In the case of being granular, the average particle diameter can be from 0.1 to 50 $\mu$m and may be from 1 to 10 $\mu$m.

The content of the zeolite may be from 10 to 75% by mass or from 15 to 60% by mass based on the total amount of the deodorizing catalyst in order to exhibit optimum deodorizing performance. From the viewpoint of deodorizing performance, the ratio of the content of the zeolite with respect to the content of the activated carbon described below (zeolite/activated carbon) may be from 0.1 to 5.0 or from 0.3 to 3.0.

(Activated Carbon)

The activated carbon can be obtained, for example, by activating the following activated carbon precursor using water vapor.

Activated carbon precursors: polyacrylonitrile, pitch, cellulose, and the like; sawdust, wood chips, wood, peat, charcoal, coconut shell, coal, carbonaceous substances (petroleum coke, coal coke, petroleum pitch, coal pitch, and coal tar pitch), and the like; synthetic resins, cellulosic fibers, composites thereof, and the like.

Among these, the activated carbon precursor may be coconut shell from the viewpoints of having a small pore diameter and excellent deodorizing performance and of having a relatively large specific gravity and good processability.

Examples of the form of the activated carbon and the activated carbon precursor include a granular form and a fibrous form. Among these, from the viewpoint of moldability, the form may be granular. In the case of being granular, the average particle diameter can be from 1 to 200 $\mu$m, and may be from 10 to 100 $\mu$m.

The specific surface area of the activated carbon can be 500 $m^2$/g or more and may be 700 $m^2$/g or more from the viewpoint of deodorizing performance. The upper limit of the specific surface area is not particularly limited, but can be, for example, 2500 $m^2$/g. The specific surface area can be determined by the BET method (multipoint method or one-point method) described in JIS K1477.

The content of the activated carbon can be from 10 to 75% by mass and may be from 15 to 60% by mass based on the total amount of the deodorizing catalyst in order to exhibit optimum deodorizing performance.

(Other Components)

The deodorizing catalyst may contain other components in addition to the above copper-manganese-based composite oxide, zeolite, and activated carbon as main components. Examples of other components include a binder for binding main components, a dispersant, and an antifoaming agent.

Examples of the binder include organic binders and inorganic binders. Examples of the organic binders include an acrylic resin, a urethane resin, a vinyl acetate resin, an SBR resin, an epoxy resin, and a polyvinyl alcohol resin. Examples of the inorganic binders include silica sol, alumina sol, and titania sol. The content of the binder can be appropriately adjusted, and can be, for example, from 3 to 50% by mass or may be from 5 to 20% by mass, based on the total amount of the deodorizing catalyst.

<Slurry for Forming Deodorizing Catalyst>

The deodorizing catalyst can be formed using a slurry for forming a deodorizing catalyst containing the above components. The deodorizing catalyst includes a copper-manganese-based composite oxide, zeolite, activated carbon,

and a liquid component.

(Liquid Component)

The liquid component may be an aqueous component, a non-aqueous component such as alcohol, acetone, or hexane, or a mixed component of these. However, from the viewpoint of dispersibility and safety of each of the above components, an aqueous component is preferable. The amount of each of the above components to be added to the liquid component is only required to be appropriately adjusted such that a deodorizing catalyst to be obtained has the above desired composition.

The content of the liquid component can be from 40 to 99% by mass based on the total amount of the slurry, from the viewpoint of a slurry viscosity suitable for molding.

<Deodorizing Catalyst Structure>

The deodorizing catalyst structure includes a substrate and a catalyst layer containing the above deodorizing catalyst on the substrate.

(Substrate)

The substrate is a so-called catalyst carrier, and the shape thereof is not particularly limited. Examples of the substrate include plate-shaped or block-shaped bulk members, members having a honeycomb structure, pellet-shaped members, and woven or nonwoven fabric-shaped members. Other examples of the substrate include constituent members of home appliances, wall surfaces of buildings, and the like.

The material of the substrate is not particularly limited, and examples thereof include metals, ceramics, glass, plastics, cellulosic materials, and materials obtained by combining these materials (composite material, laminated material, and the like).

Examples of the metals include stainless steel, aluminum, copper, galvanized steel sheets, and iron. Examples of the ceramics include cordierite, alumina, barium titanate, boron nitride, and silicon nitride. Examples of the glass include ordinary soda-lime glass, borosilicate glass, alkali-free glass, quartz glass, and aluminosilicate glass. Examples of the plastics include acrylic resins such as polymethyl methacrylate, aromatic polycarbonate resins such as polyphenylene carbonate, and aromatic polyester resins such as polyethylene terephthalate (PET). Examples of the cellulosic materials include cotton, hemp, rayon, and cupra.

(Catalyst Layer)

The catalyst layer is formed using the above slurry for forming a deodorizing catalyst. That is, the catalyst layer includes a copper-manganese-based composite oxide, zeolite, and activated carbon. The amount to be carried on the catalyst layer varies depending on the substrate to be used. For example, when a honeycomb substrate is used, the amount to be carried can be set to from 30 to 300 g/L in order for the catalyst to exert sufficient deodorizing performance. In addition, the thickness of the catalyst layer varies depending on the substrate to be used. For example, when a honeycomb substrate is used, the thickness can be set to from 10 to 300 $\mu$m, from the viewpoint of suppressing delamination of the catalyst layer.

<Method for Producing Deodorizing Catalyst Structure>

The method for producing a deodorizing catalyst structure includes a step of forming a catalyst layer on a substrate using the above slurry for forming a deodorizing catalyst. Specifically, the method for producing can comprise, for example, a step of applying a slurry for forming a deodorizing catalyst to a substrate (application step), and a step of removing a liquid component from the applied slurry for forming a deodorizing catalyst (removal step).

(Application Step)

An application method is not particularly limited, and examples thereof include a washcoating method, a spin coating method, a dip coating method, a spray coating method, a flow coating method, a bar coating method, and a gravure coating method. When a honeycomb substrate is used, a common washcoating method is suitable. These application methods may be used singly or in combination of two or more kinds thereof.

(Removal Step)

A removal method is not particularly limited, and examples thereof include a method of leaving the substrate after application of the slurry for forming a deodorizing catalyst at room temperature, a method of blowing gas onto the substrate, and a method of heating the substrate to a predetermined temperature. The heating temperature in the heating method depends on the heat resistance of the substrate, and can be, for example, 100°C or higher. These removal methods may be used singly or in combination of two or more kinds thereof. Through this step, a catalyst layer containing a deodorizing catalyst containing the above components is formed on the surface of the substrate.

<Deodorization Method>

The deodorization method includes a step of bringing the above deodorizing catalyst or the above deodorizing catalyst structure into contact with an odor containing a sulfur compound and an amine-based compound (deodorizing step).

The environment for the deodorizing step can be set to a temperature of from 0 to 40°C and a relative humidity of from 1 to 90% in order for the catalyst to exert sufficient deodorizing performance. The deodorizing catalyst or the deodorizing catalyst structure can be place in, for example, a refrigerator, an air cleaner, an air conditioner, a ventilation fan, or the like.

Examples

The present disclosure will be described in more detail with reference to the following examples, but the present disclosure is not limited to these examples.

<Preparation of Slurry for Forming Deodorizing Catalyst>

(Example 1)

The following components were added to 100.0 g of ion exchanged water with stirring using a propeller stirrer. After the addition, the mixture was stirred for 30 minutes to obtain a slurry.

· 13.0 g of Aron NS-1200 (acrylic binder, solid concentration: 40%) manufactured by Toagosei Co., Ltd.
· 11.1 g of LZY-84 (Y-type zeolite, silica/alumina molar ratio = 5, cationic species = hydrogen ion, particle size = 6.9 $\mu$m, solid concentration: 85.0%) manufactured by UOP
· 29.4 g of KURARAY COAL PGW-20MP (activated carbon, specific surface area: 930 m$^2$/g, particle size = 18.1 $\mu$m, solid concentration: 95.9%) manufactured by Kuraray Co., Ltd.
· 7.4 g of DAIPYROXIDE #7700 (Cu Mn composite oxide, particle size = 17.8 $\mu$m, solid concentration: 97.3%) manufactured by Dainichiseika Color & Chemicals Mfg. Co., Ltd.

(Example 2)

A slurry was obtained in the same manner as in Example 1 except that the amount of LZY-84 added was 22.1 g and the amount of KURARAY COAL PGW-20MP added was 19.6 g.

(Example 3)

A slurry was obtained in the same manner as in Example 1 except that the amount of LZY-84 added was 33.2 g and the amount of KURARAY COAL PGW-20MP added was 9.8 g.

(Example 4)

A slurry was obtained in the same manner as in Example 1 except that the amount of LZY-84 added was 18.0 g, the amount of KURARAY COAL PGW-20MP added was 15.9 g, and the amount of DAIPYROXIDE #7700 added was 14.7 g.

(Example 5)

A slurry was obtained in the same manner as in Example 1 except that the amount of LZY-84 added was 22.1 g, the amount of KURARAY COAL PGW-20MP added was 19.6 g, and 8.1 g of N-840P (Cu Mn composite oxide, particle size = 6.6 $\mu$m, solid concentration: 88.0%) manufactured by Clariant Catalyst Co., Ltd. was added instead of DAIPYROXIDE #7700.

(Example 6)

A slurry was obtained in the same manner as in Example 1 except that the amount of LZY-84 added was 22.1 g and 19.6 g of UCG-MD (activated carbon, specific surface area: 1380 m$^2$/g, particle size = 16.5 $\mu$m, solid concentration: 95.9%) manufactured by UES Co., Ltd. was added instead of KURARAY COAL PGW-20MP

(Example 7)

A slurry was obtained in the same manner as in Example 1 except that the amount of LZY-84 added was 22.1 g and 19.0 g of Triporous (activated carbon, specific surface area: 760 m$^2$/g, particle size = 86.8 $\mu$m, solid concentration: 99.0%) manufactured by Sony Group Corporation was added instead of KURARAY COAL PGW-20MP

(Example 8)

A slurry was obtained in the same manner as in Example 1 except that the amount of LZY-84 added was 22.1 g and 19.0 g of high specific surface area activated carbon (activated carbon, specific surface area: 2130 m$^2$/g, particle size = 26.6 $\mu$m, solid concentration: 99.0%) manufactured by manufactured by Kuraray Co., Ltd. was added instead of KURARAY COAL PGW-20MP

(Example 9)

A slurry was obtained in the same manner as in Example 1 except that 21.4 g of LZY-54 (Y-type zeolite, silica/alumina molar ratio = 5, cationic species = sodium, particle size = 4.4 $\mu$m, solid concentration: 88.4%) manufactured by UOP was added instead of LZY-84 and the amount of KURARAY COAL PGW-20MP added was 19.6 g.

(Example 10)

A slurry was obtained in the same manner as in Example 1 except that 19.0 g of HSZ-390HUA (Y-type zeolite, silica/alumina molar ratio = 770, cationic species = hydrogen ion, particle size = 6.0 $\mu$m, solid concentration: 99.0%) manufactured by Tosoh Corporation was added instead of LZY-84 and the amount of KURARAY COAL PGW-20MP added was 19.6 g.

(Example 11)

A slurry was obtained in the same manner as in Example 1 except that 18.8 g of HSZ-320HOA (Y-type zeolite, silica/alumina molar ratio = 6, cationic species = hydrogen ion, particle size = 7.0 $\mu$m, solid concentration: 99.9%) manufactured by Tosoh Corporation was added instead of LZY-84 and the amount of KURARAY COAL PGW-20MP added was 19.6 g.

(Example 12)

A slurry was obtained in a similar manner to Example 1 except that 19.0 g of HSZ -3 50HUA (Y-type zeolite, silica/alumina molar ratio = 11, cationic species = hydrogen ion, particle size = 6.0 $\mu$m, solid concentration 99.0%) manufactured by Tosoh Corporation was added instead of LZY-84 and the amount of KURARAY COAL PGW-20MP added was 19.6 g.

(Example 13)

A slurry was obtained in the same manner as in Example 1 except that 18.8 g of HSZ-360HUA (Y-type zeolite, silica/alumina molar ratio = 15, cationic species = hydrogen ion, particle size = 6.0 $\mu$m, solid concentration 99.9%) manufactured by Tosoh Corporation was added instead of LZY-84 and the amount of KURARAY COAL PGW-20MP added was 19.6 g.

(Comparative Example 1)

A slurry was obtained in a similar manner to Example 1 except that LZY-84 was not used and the amount of KURARAY COAL PGW-20MP added was 39.3 g.

(Comparative Example 2)

A slurry was obtained in the same manner as in Example 1 except that KURARAY COAL PGW-20MP was not used and the amount of LZY-84 added was 44.3 g.

(Comparative Example 3)

A slurry was obtained in the same manner as in Example 1 except that LZY-84 and KURARAY COAL PGW-20MP were not used and the amount of DAIPYROXIDE #7700 added was 46.0 g.

(Comparative Example 4)

A slurry was obtained in the same manner as in Example 1 except that the amount of LZY-84 added was 22.1 g, the amount of KURARAY COAL PGW-20MP added was 19.6 g, and 7.5 g of activated manganese dioxide 250 ($MnO_2$, particle size = 0.6 $\mu$m, solid concentration: 95.0%) manufactured by Japan Metals & Chemicals Co., Ltd. was added instead of DAIPYROXIDE #7700.

(Comparative Example 5)

A slurry was obtained in the same manner as in Example 1 except that the amount of LZY-84 added was 22.1 g, the amount of KURARAY COAL PGW-20MP added was 19.6 g, and 7.5 g of CARULITE 400 Type E ($MnO_2$, particle size = 4.0 $\mu$m, solid concentration: 95.4%) manufactured by Carus was added instead of DAIPYROXIDE #7700.

(Comparative Example 6)

A slurry was obtained in the same manner as in Example 1 except that the amount of LZY-84 added was 22.1 g, the amount of KURARAY COAL PGW-20MP added was 19.6 g, and 7.4 g of activated manganese dioxide LMD181 ($MnO_2$, particle size = 7.0 $\mu$m, solid concentration: 96.0%) manufactured by Japan Metals & Chemicals Co., Ltd. was added instead of DAIPYROXIDE #7700.

(Comparative Example 7)

A slurry was obtained in the same manner as in Example 1 except that the amount of LZY-84 added was 22.1 g, the amount of KURARAY COAL PGW-20MP added was 19.6 g, and 6.4 g of activated manganese dioxide 250 and 1.1 g of copper oxide NB -2 (CuO, particle size = 2.4 $\mu$m, solid concentration: 99.6%) manufactured by Nisshin Chemco Ltd. were added instead of DAIPYROXIDE #7700.

<Production of Deodorizing Filter>

A rayon paper honeycomb (number of cells: 200/inch$^2$, cell opening surface diameter: 21 mm, or cell opening surface: 22 mm $\times$ 23 mm, length in ventilation direction: 6.5 mm) manufactured by Shanghai Azumi Daido Filter Co., Ltd. was provided as a honeycomb substrate. After this was immersed in the slurry of each example, the excess slurry was removed by air blowing. Thereafter, the substrate was placed in a dryer set at 150°C for 1 hour and dried to obtain a deodorizing filter (deodorizing catalyst structure). The washcoat amount (coating layer amount) after drying was 100 g per 1 L of the honeycomb substrate.

<Evaluation>

Deodorizing performance tests (1) and (2) were performed using the deodorizing filter of each example. The results are shown in Table 1.

Test (1): Deodorizing performance on methyl mercaptan

A deodorizing filter carrying a honeycomb substrate and having a cell opening surface diameter of 21 mm was placed in a glass tube. Air containing 100 ppm methyl mercaptan (MMP) at a temperature of 25°C and a humidity of 18% RH was allowed to pass through the deodorizing filter at a flow rate of 9.7 L/min for 30 minutes. The methyl mercaptan concentration after 30 minutes was measured at the catalyst outlet, and the removal rate after 30 minutes was calculated by the following formula.

$$\text{MMP removal rate (\%)} = \{1 - (\text{MMP concentration after 30 minutes [ppm]}/100 \text{ [ppm]})\} \times 100$$

Test (2): Deodorizing performance on trimethylamine

A ventilator including a deodorizing filter carrying a honeycomb substrate and having a cell opening surface of 22 mm × 23 mm set therein was placed in a glass container having a volume of 30 L. The temperature and the humidity in the container were adjusted to 25°C and to 50% RH, respectively. Trimethylamine (TMA) having a concentration corresponding to 100 ppm was injected into the container and circulated through the deodorizing filter at a linear velocity of 0.5 m/s for 30 minutes. The trimethylamine concentration after 30 minutes was measured, and the removal rate after 30 minutes was calculated by the following formula.

$$\text{TMA removal rate (\%)} = \{1 - (\text{TMA concentration after 30 minutes [ppm]}/100 \text{ [ppm]})\} \times 100$$

[Table 1-1]

| | | Example 1 | Example 2 | Example 3 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|---|
| Composition [% by mass] | N-840P | - | - | - | - | 14.3 | - | - | - | - | - | - | - | - |
| | DAIPYROXIDE #7700 | 14.3 | 14.3 | 14.3 | 28.6 | - | 143 | 143 | 143 | 143 | 143 | 143 | 143 | 14.3 |
| | LZY-84 | 18.8 | 37.6 | 56.4 | 30.5 | 37.6 | 37.6 | 37.6 | 37.6 | - | - | - | - | - |
| | LZY-54 | - | - | - | - | - | - | - | - | 37.6 | - | - | - | - |
| | HSZ-390HUA | - | - | - | - | - | - | - | - | - | 37.6 | - | - | - |
| | HSZ-320HOA | - | - | - | - | - | - | - | - | - | - | 37.6 | - | - |
| | HSZ-350HUA | - | - | - | - | - | - | - | - | - | - | - | 37.6 | - |
| | HSZ-360HUA | - | - | - | - | - | - | - | - | - | - | - | - | 37.6 |
| | PGW-20MP | 56.4 | 37.6 | 18.8 | 30.5 | 37.6 | - | - | - | 37.6 | 37.6 | 37.6 | 37.6 | 37.6 |
| | UCG-MD | - | - | - | - | - | 37.6 | - | - | - | - | - | - | - |
| | Triporous | - | - | - | - | - | - | 37.6 | - | - | - | - | - | - |
| | High specific surface area activated carbon | - | - | - | - | - | - | - | 37.6 | - | - | - | - | - |
| | NS-1200 | 10.4 | 10.4 | 10.4 | 10.4 | 10.4 | 10.4 | 10.4 | 10.4 | 10.4 | 10.4 | 10.4 | 10.4 | 10.4 |
| | Total | 100 | 100 | 100 | 100 | 100 | 100 | 100 | 100 | 100 | 100 | 100 | 100 | 100 |
| Removal rate after 30 minutes | MMP removal rate | 60% | 58% | 53% | 71% | 69% | 61% | 58% | 56% | 53% | 54% | 53% | 51% | 51% |
| | TMA removal rate | 89% | 93% | 91% | 90% | 92% | 90% | 92% | 91% | 87% | 86% | 95% | 96% | 92% |

EP 4 029 533 A1

[Table 1-2]

| | | Comparative Example | Comparative Example | Comparative Example | Comparative Example | Comparative Example | Comparative Example | Comparative Example |
|---|---|---|---|---|---|---|---|---|
| | | 1 | 2 | 3 | 4 | 5 | 6 | 7 |
| Composition [% by mass] | DAIPYROXIDE #7700 | 143 | 14.3 | 89.6 | - | - | - | - |
| | Activated manganese dioxide 250 | - | - | - | 14.3 | - | - | 12.2 |
| | CARULITE400 Type E | - | - | - | - | 14.3 | - | - |
| | Activated manganese dioxide LMD181 | - | - | - | - | - | 14.3 | - |
| | Copper oxide NB-2 | - | - | - | - | - | - | 2.1 |
| | LZY-84 | - | 75.3 | - | 37.6 | 37.6 | 37.6 | 37.6 |
| | PGW-20MP | 753 | - | - | 37.6 | 37.6 | 37.6 | 37.6 |
| | NS-1200 | 10.4 | 10.4 | 10.4 | 10.4 | 10.4 | 10.4 | 10.4 |
| | Total | 100 | 100 | 100 | 100 | 100 | 100 | 100 |
| Removal rate after 30 minutes | MMP removal rate | 42% | 29% | 63% | 49% | 33% | 29% | 48% |
| | TMA removal rate | 89% | 92% | 72% | 85% | 86% | 86% | 92% |

As a supplementary experiment, a deodorizing performance test (3) was performed using zeolite singly. The results are shown in Table 2.

Test (3): Powder trimethylamine removal test

A watch glass with 0.2 g of zeolite powder was placed in a glass container having a volume of 30 L. The temperature and the humidity in the container were adjusted to 25°C and to 50% RH, respectively. Trimethylamine (TMA) corresponding to a concentration of 100 ppm was injected into the container, and the stirring fan was turned on for 15 minutes. The trimethylamine concentration after 15 minutes was measured, and the removal rate after 15 minutes was calculated by the following formula.

$$\text{TMA removal rate (\%)} = \{1 - (\text{TMA concentration after 15 minutes [ppm]}/100\ [\text{ppm}])\} \times 100$$

[Table 2]

| Zeolite name | Silica/alumina molar ratio | Structure (type) | Cationic species | TMA removal rate after 15 minutes | Remarks |
|---|---|---|---|---|---|
| Mizuka sieves Y-420 | 4 | Y | $H^+$ | 75% | - |
| LZY-84 | 5 | Y | $H^+$ | 82% | Used in Example 1 |
| HSZ-320HOA | 6 | Y | $H^+$ | 76% | Used in Example 11 |
| NFK-7-2SC1_0-HWOS | 6 | Y | $H^+$ | 76% | - |
| HSZ-350HUA | 11 | Y | $H^+$ | 84% | Used in Example 12 |
| HSZ-640HOA | 18 | MOR | $H^+$ | 82% | - |
| NFK-5D-25HX | 28 | MFI | $H^+$ | 84% | - |
| Zibo | 28 | MFI | $H^+$ | 78% | - |
| HSZ-385HUA | 115 | Y | $H^+$ | 65% | - |
| NU-2080 | 375 | MFI | $H^+$ | 69% | - |
| HSZ-390HUA | 770 | Y | $H^+$ | 60% | Used in Example 10 |
| BLANK | - | - | - | 34% | - |

The details of the zeolites in Table 2 are as follows.

Mizuka sieves Y -420: manufactured by Mizusawa Industrial Chemicals, Ltd.
NFK-7-2SC1_0-HWOS: manufactured by Nankai University Catalyst
HSZ-640HOA: manufactured by Tosoh Corporation
NFK-5D-25HX: manufactured by Nankai University Catalyst
Zibo: manufactured by Zibo Xinhong Chemical Trading
HSZ-385HUA: manufactured by Tosoh Corporation
NU-2080: manufactured by UOP

**Claims**

1. A deodorizing catalyst comprising: a copper-manganese-based composite oxide; zeolite; and activated carbon.

2. The deodorizing catalyst according to claim 1, comprising: 10 to 50% by mass of the copper-manganese-based composite oxide; 10 to 75% by mass of the zeolite; and 10 to 75% by mass of the activated carbon, based on a total amount of the deodorizing catalyst.

3. The deodorizing catalyst according to claim 1 or 2, wherein the zeolite has a silica/alumina molar ratio of 100 or less.

4. The deodorizing catalyst according to any one of claims 1 to 3, wherein a cationic species of the zeolite is a hydrogen ion.

5. The deodorizing catalyst according to any one of claims 1 to 4, wherein the activated carbon has a specific surface area of 500 $m^2$/g or more.

6. The deodorizing catalyst according to any one of claims 1 to 5, wherein the deodorizing catalyst is for an odor including a sulfur compound and an amine-based compound.

7. A deodorizing catalyst structure comprising: a substrate; and a catalyst layer containing the deodorizing catalyst according to any one of claims 1 to 6 on the substrate.

8. A deodorization method comprising: a step of bringing the deodorizing catalyst according to any one of claim 1 to 6 or the deodorizing catalyst structure according to claim 7 into contact with an odor containing a sulfur compound and an amine-based compound.

9. A slurry for forming a deodorizing catalyst comprising: a copper-manganese-based composite oxide; zeolite; activated carbon; and a liquid component.

10. A method for producing a deodorizing catalyst structure, comprising: a step of forming a catalyst layer on a substrate using the slurry according to claim 9.

## INTERNATIONAL SEARCH REPORT

| | International application No. |
|---|---|
| | PCT/JP2019/050962 |

**A. CLASSIFICATION OF SUBJECT MATTER**
Int. Cl. A61L9/01(2006.01)i, A61L9/014(2006.01)i, B01J23/72(2006.01)i,
B01J29/08(2006.01)i
FI: A61L9/01 E, A61L9/014, B01J29/08 M, B01J23/72 M
According to International Patent Classification (IPC) or to both national classification and IPC

**B. FIELDS SEARCHED**

Minimum documentation searched (classification system followed by classification symbols)
Int. Cl. A61L9/01, A61L9/014, B01J23/72, B01J29/08

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 9-299461 A (MATSUSHITA ELECTRIC INDUSTRIAL CO., | 1-4, 6-8 |
| Y | LTD.) 25 November 1997, claims, paragraphs [0005]-[0009], [0029], [0044], examples | 3-10 |
| X | JP 9-47500 A (KOBE STEEL, LTD.) 18 February 1997, | 1-3, 7, 9-10 |
| Y | claims, paragraphs [0012], [0015]-[0017], [0020], [0021], examples 1, 15 | 4-10 |
| X | JP 10-99690 A (SANYO ELECTRIC CO., LTD.) 21 April | 1-4 |
| Y | 1998, claims, paragraphs [0010], [0011], [0016], [0020], [0022], [0026], [0037], examples | 5-10 |
| X | JP 2007-175085 A (SNT CO.) 12 July 2007, claims, | 1-2, 6, 8 |
| Y | paragraph [0021], example 2 | 3-6, 8 |
| A | | 7, 9-10 |

☒ Further documents are listed in the continuation of Box C.　　☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12.02.2020 | 25.02.2020 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
|---|
| PCT/JP2019/050962 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 10-33646 A (NIPPON SHOKUBAI CO., LTD.) 10 February 1998, claims, paragraphs [0004], [0013] | 5-8 |
| Y | JP 6-254140 A (BABCOCK-HITACHI KABUSHIKI KAISHA) 13 September 1994, paragraphs [0005]-[0009] | 3-8 |
| A | JP 2001-207504 A (MATSUSHITA ELECTRIC INDUSTRIAL CO., LTD.) 03 August 2001, entire text, all drawings | 1-10 |
| A | JP 2009-279522 A (KOBE STEEL, LTD.) 03 December 2009, entire text, all drawings | 1-10 |
| A | JP 10-180108 A (MATSUSHITA ELECTRIC INDUSTRIAL CO., LTD.) 07 July 1998, entire text, all drawings | 1-10 |

Form PCT/ISA/210 (continuation of second sheet) (January 2015)

**INTERNATIONAL SEARCH REPORT**
Information on patent family members

International application No.

PCT/JP2019/050962

| Patent Documents referred to in the Report | Publication Date | Patent Family | Publication Date |
|---|---|---|---|
| JP 9-299461 A | 25.11.1997 | (Family: none) | |
| JP 9-47500 A | 18.02.1997 | (Family: none) | |
| JP 10-99690 A | 21.04.1998 | (Family: none) | |
| JP 2007-175085 A | 12.07.2007 | (Family: none) | |
| JP 10-33646 A | 10.02.1998 | (Family: none) | |
| JP 6-254140 A | 13.09.1994 | (Family: none) | |
| JP 2001-207504 A | 03.08.2001 | (Family: none) | |
| JP 2009-279522 A | 03.12.2009 | (Family: none) | |
| JP 10-180108 A | 07.07.1998 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H10137591 A